# EUROPEAN PATENT APPLICATION

(11) **EP 3 620 151 A1**
(43) Date of publication of application: **11.03.2020**
(21) Application number: 18789896.0
(22) Date of filing: 19.04.2018
(51) Int. Cl.: A61K 8/64, A61K 8/44, A61K 8/46, A61Q 5/00, D06M 13/252, D06M 15/15

(54) **FIBER TREATMENT AGENT AND METHOD FOR USING SAME**

(30) Priority: 25.04.2017 JP 2017086082
(71) Applicant: Little Scientist Co., Ltd., Ichinomiya-shi, Aichi 494-0001 (JP)
(72) Inventor: NOMURA, Yasutoshi, Ichinomiya-shi Aichi 494-0001 (JP); TSUBOI, Takayuki, Ichinomiya-shi Aichi 494-0001 (JP)
(74) Representative: Office Freylinger
(86) International application number: PCT/JP2018/016182
(87) International publication number: WO 2018/198942

(57) **Abstract**

Provided is a fiber treatment agent which exhibits excellent adhesion to an application site and is capable of improving, protecting, or repairing functions and characteristics of an application site and a method for using the same.

According to the present invention, there is provided a fiber treatment agent, in which: the fiber treatment agent is prepared in a one-component formulation containing an effective amount of a reducing agent and an effective amount of a converted protein in which some or all of disulfide bonds (-S-S-) in a protein are converted by at least one of a thiol compound selected from the group consisting of cysteamine, cysteine, and thioglycolic acid or sulfurous acid, or prepared in a two-component formulation including a first agent containing the converted protein and a second agent containing the reducing agent, in which a total content of the converted proteins and a total content of the reducing agents in the first agent and the second agent satisfy the respective effective amounts; and the fiber treatment agent is used for improving strength of a fiber or improving irregularity on a hair end caused by a flipped-up or wavy fiber.

## Description

### TECHNICAL FIELD

The present invention relates to a fiber treatment agent and a method for using the same, more specifically, a fiber treatment agent which exhibits excellent adhesion to an application site and is capable of improving, protecting, or repairing functions and characteristics of an application site and a method for using the same.

### BACKGROUND ART

A fiber treatment agent is used not only for making the appearance beautiful and improving the touch of a fiber, but sometimes for protecting an application site, or improving or repairing functions and characteristics of an application site. As a component contained in such a fiber treatment agent, a protein is conventionally known. For example, keratin, which is a protein contained in a feather, a hair, and the like, is known to have a high affinity to a feather, silk, cotton, skin, and a hair and be effective in moisturizing and improving the touch of a fiber such as a hair. Proteins are varied in type, structure, and characteristic, and, in particular, some proteins such as keratin form a large number of intramolecular or intermolecular disulfide bonds (-S-S-).

A protein is usually insoluble to an oil-based solvent. Further, a protein having a disulfide bond is also insoluble to water. Thus, such a disulfide bond is conventionally cleaved by reduction to form a thiol group (-SH) or a derivative group thereof, thereby solubilizing the protein in water. For example, Patent Literature 1 describes a cosmetic in which a peptide having -SS-(CH₂)ₙCOO- as a side chain group is compounded. Patent Literature 2 describes a water-solubilized protein obtained by reducing disulfide bonds in a water-insoluble protein to thiol groups and converting some or all of the thiol groups to carboxymethyl disulfide groups (-SSCH₂COOH).

As a protein used for a fiber treatment, a protein in which cysteine is converted to cysteic acid by oxidatively decomposing a disulfide bond for increasing water solubility is also known. However, a disulfide bond is lost in this protein.

### CITATIONS LIST

### PATENT LITERATURE

Patent Literature 1: JP 2010-285401 A
Patent Literature 2: JP H07-126296 A

### SUMMARY OF INVENTION

### TECHNICAL PROBLEMS

However, when a fiber treatment agent is used, the fiber treatment agent is required not only being soluble in a certain solvent, but also to have excellent adhesion to an application site (e.g., a hair). Normally, in a method for treating a fiber using a protein, proteins adhered to the surface or the inside of the fiber are gradually eluted by cleaning, washing, or the like, making it difficult to maintain sustainable effects.

An object of the present invention is to provide a fiber treatment agent which exhibits excellent adhesion to an application site and is capable of improving the strength of a fiber or irregularity on a hair end caused by a flipped-up or wavy fiber, and a method for using the fiber treatment agent.

### SOLUTIONS TO PROBLEMS

According to the present invention, there is provided a fiber treatment agent, in which: the fiber treatment agent is prepared in a one-component formulation containing an effective amount of a reducing agent and an effective amount of a converted protein in which some or all of disulfide bonds (-S-S-) in a protein are converted by at least one of a thiol compound selected from the group consisting of cysteamine, cysteine, and thioglycolic acid or sulfurous acid, or prepared in a two-component formulation including a first agent containing the converted protein and a second agent containing the reducing agent, in which a total content of the converted proteins and a total content of the reducing agents in the first agent and the second agent satisfy the respective effective amounts; and the fiber treatment agent is used for improving strength of a fiber or improving irregularity on a hair end caused by a flipped-up or wavy fiber.

According to the present invention, there is provided a method for using the fiber treatment agent, in which the fiber treatment agent is applied to a fiber and then an oxidizing agent is applied to the fiber.

### ADVANTAGEOUS EFFECTS OF INVENTION

The fiber treatment agent of the present invention, which exhibits excellent adhesion to an application site such as a hair, can protect or repair the application site, or improve functions or characteristics of the application site.

### BRIEF DESCRIPTION OF DRAWINGS

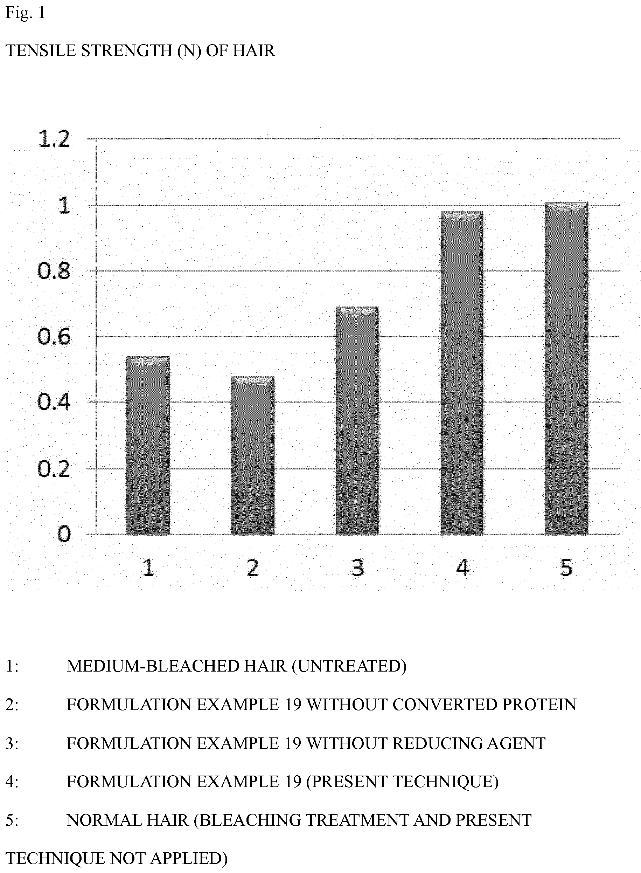

Fig. 1 is a diagram illustrating a measurement result of the strength of a hair after being treated by a fiber treatment agent.

### DESCRIPTION OF EMBODIMENTS

No particular limitation is imposed on the type, origin, and structure of the protein described above as long as it contains a disulfide bond. As the protein, for example, a water-insoluble protein can be used. Specific examples of the water-insoluble protein include keratin, casein, collagen, elastin, and a soybean protein. The protein may be purified and isolated to use, or a natural component containing the protein may be used. Examples of the natural component include a hair containing keratin derived from human, a bird, or an animal (human hair, wool, feather), a horn, and a nail. In one embodiment of the fiber treatment agent of the present invention, as the protein, keratin obtained from wool or a feather can be used.

The disulfide bond may be formed within one protein molecule or may be formed between two or more protein molecules.

The converted protein described above is a protein in which some or all of disulfide bonds in the protein described above are converted by at least one of a thiol compound selected from the group consisting of cysteamine, cysteine, and thioglycolic acid or sulfurous acid (hereinafter referred to as "thiol compound or the like"). The term "conversion" means that a disulfide bond in the protein is cleaved and then an S atom at the cleavage site and an S atom in the thiol compound or the like form a new bond (see the following Formulas (1) and (2); in Formulas, R varies depending on the type of compounds). As described above, in the present invention, some of disulfide bonds in the protein may be converted by the thiol compound or the like. Thus, the converted protein may contain remaining unconverted disulfide bonds or thiol groups formed by the cleavage of disulfide bonds. The converted protein may be used singly, or in combination of two or more kinds thereof.

Depending on the amount of the thiol compound or the like and other reaction conditions, there are the case (the following Formula (1)) where one end of the disulfide bond is converted by the thiol compound or the like and the other end is converted to a thiol group and the case (the following Formula (2)) where both ends of the disulfide bond are converted by the thiol compounds or the like. The above term "conversion" includes both cases. Note that, in the following Formulas (1) and (2), "Cys" means a cysteine residue contained in the protein.
(Chemical 1)

Cys-S-S-Csy + R-SH -> Cys-S-S-R + Cys-SH (1)

(Chemical 2)

Cys-S-S-Csy + H₂N-CHR-CH₂-SH -> 2 Csy-S-S-CH₂-CHR-NH₂ (2)

The converted protein may be subjected to other treatments as needed. For example, in order to lower the molecular weight and molecular size, the water-insoluble protein and/or the converted protein may be hydrolyzed with an enzyme, an acid, or an alkali. Further, after being obtained, the converted protein may be subjected to purification, filtration, centrifugal separation, or the like to remove other components or insoluble matters.

In the fiber treatment agent of the present invention, the content of the converted protein is not particularly limited as long as it satisfies an effective amount. The content of the converted protein is usually 0.01 to 10% by mass.

The reducing agent can reduce a disulfide bond contained in the converted protein and/or a fiber such as a hair to be treated and convert the disulfide bond to a thiol group. The type of the reducing agent is not particularly limited as long as the conversion can be achieved. Specific examples of the usable reducing agent include thioglycolic acid, thiolactic acid, sulfurous acid, cysteamine, cysteine, spironolactone, and a salt or derivative (e.g., ester) of each of these reducing agents. The reducing agent may be used singly, or in combination of two or more kinds thereof.

In the fiber treatment agent of the present invention, the content of the reducing agent is not particularly limited as long as it satisfies an effective amount. The content of the reducing agent is usually 0.01 to 12% by mass.

The fiber treatment agent of the present invention may be prepared in a one-component formulation containing effective amounts of both the converted protein and the reducing agent. Further, the fiber treatment agent of the present invention may be prepared in a two-component formulation including a first agent containing an effective amount of the converted protein and a second agent containing an effective amount of the reducing agent. In the case of the two-component formulation, no particular limitation is imposed on specific compositions of the first agent and the second agent. The compositions of the first agent and the second agent may be the same or different from each other. Further, in the case of the two-component formulation, the converted protein may be contained only in the first agent or both in the first agent and the second agent. The reducing agent may be contained only in the second agent or both in the first agent and the second agent.

In the case of the two-component formulation, the total content of the converted proteins and the total content of the reducing agents in the first agent and the second agent satisfy the respective effective amounts. For example, in one embodiment of the fiber treatment agent of the present invention, the converted protein is contained only in the first agent in the effective amount. Further, in another embodiment of the fiber treatment agent of the present invention, the converted protein is contained both in the first agent and the second agent in an amount less than the effective amount with the total content of both satisfying the effective amount. This description is also applied to the reducing agent.

The fiber treatment agent of the present invention can improve the strength of a fiber or improve irregularity on a hair end caused by a flipped-up or wavy fiber (e.g., a state in which a short hair sticks out from the surface of dressed hairs, so called "ahoge"). The phrase of "improving" the strength of a fiber means both restoring the lowered strength and further increasing the strength. Further, the phrase of "improving" irregularity on a hair end caused by a flipped-up or wavy fiber means both eliminating irregularity completely to dress hairs and reducing a degree of irregularity.

The fiber treatment agent of the present invention may contain other components than the converted protein and the reducing agent as needed, as long as action effects of the present invention are not inhibited. Examples of other components include a known component which is conventionally added and incorporated in a fiber treatment agent or other functional components. Specific examples of other components include fat and oil, hydrocarbon oil, a higher fatty acid, a higher alcohol, ester oil, silicone oil, an anionic surfactant (anionic, cationic, amphoteric, nonionic), a humectant, a water-soluble polymer, a coating agent, a ultraviolet absorber, a sequestering agent, a lower alcohol, a polyhydric alcohol, a saccharide, an amino acid, a pH adjuster, a vitamin, an antioxidant, a dye, a preservative, and a perfume.

No particular limitation is imposed on a dosage form and a mode of use of the fiber treatment agent of the present invention. The fiber treatment agent of the present invention can be used, for example, as lotion, emulsion, gel, ointment/wax, foam, solid/powder, or mist.

No particular limitation is imposed on an application site of the fiber treatment agent of the present invention. For example, the fiber treatment agent of the present invention can be used as a cosmetic, for example, a cosmetic applied to a hair. That is, the fiber treatment agent of the present invention can be used for improving, protecting, or repairing a hair. Further, the fiber treatment agent of the present invention may be used for a hair of an animal other than human, silk, and a fiber having a disulfide bond or a thiol group such as a cotton thread.

A specific application of the fiber treatment agent of the present invention is not limited, as long as it is used for improving the strength of a fiber or improving irregularity on a hair end caused by a flipped-up or wavy fiber. In one embodiment of the fiber treatment agent of the present invention, the water solubility of the protein can be improved by the conversion. Thus, examples of the embodiment of the fiber treatment agent of the present invention include a cosmetic, for example, a hair cosmetic such as a shampoo, a rinse, a treatment, a conditioner, a hairdressing agent, a permanent waving agent, a curling agent, a hair coloring agent, a hair growth/hair tonic agent, and a treatment agent used before or after using these agents. Further, examples of the embodiment of the fiber treatment agent of the present invention include a softener, a water repellent, a modifier, a cleaning agent, a repairing agent, and an antistatic agent for a fiber such as, for example, a silk thread, a cotton thread, a wool thread, a cashmere thread, and an Angora thread. As described above, keratin can be preferably mentioned as the protein. Thus, as the embodiment of the fiber treatment agent of the present invention, a cosmetic, in particular, a hair cosmetic, containing keratin as the protein can be preferably mentioned.

In a method for using the fiber treatment agent of the present invention, the fiber treatment agent of the present invention is applied to a fiber and then an oxidizing agent is applied to the fiber. A disulfide bond in the fiber and a disulfide bond in the converted protein are both reduced to thiol groups by the present fiber treatment agent. Applying the oxidizing agent quickly forms a disulfide bond between the thiol group in the fiber and the thiol group in the converted protein, allowing the converted protein to be firmly adhered to the fiber.

No particular limitation is imposed on the type, origin, and structure of the oxidizing agent as long as a disulfide bond can be formed between thiol groups. Specific examples of the oxidizing agent include hydrogen peroxide, bromic acid and a salt thereof, chloric acid and a salt thereof, ozone, and a peroxide. The oxidizing agent may be used singly, or in combination of two or more kinds thereof.

As described above, no particular limitation is imposed on a dosage form and a mode of use of the fiber treatment agent of the present invention. Thus, the term "application" in the method of the present invention includes any mode of application that allows the fiber treatment agent of the present invention and the oxidizing agent to be adhered to a fiber. For example, the above term "application" not only includes a mode of application in which a liquid product is adhered to a fiber, but also includes a mode of application in which a solid product or a semisolid product is adhered to a fiber and a mode of application in which adhesion to a fiber is performed by spraying. Further, a specific method of the "application" is not particularly limited as long as the fiber treatment agent of the present invention and the oxidizing agent can be adhered to a fiber. Further, in the case where the fiber treatment agent of the present invention is prepared in a two-component formulation, a first agent and a second agent may be mixed first and then applied to a fiber. Alternatively, a first agent may be applied to a fiber first and then a second agent may be applied to the fiber.

As described above, no particular limitation is imposed on an application site of the fiber treatment agent of the present invention. Thus, specific examples of the fiber include a hair of human, a hair of an animal other than human, silk, and a fiber having a disulfide bond or a thiol group such as a cotton thread.

### EXAMPLES

Hereinafter, the present invention will be specifically described by way of Examples. Note that the present invention is not limited to embodiments illustrated in Examples. The embodiments of the present invention can be variously modified within the scope of the present invention depending on the purpose, use, and the like. It is also noted that any discussion of the results in Examples is merely an opinion of the inventor and is by no means a description intended for defining the present invention.

### (1) Formulation examples of fiber treatment agent

Specific compositions of Formulation examples 1 to 20 of the fiber treatment agent are shown below.

### (Formulation Example 1) Fiber cleaning agent

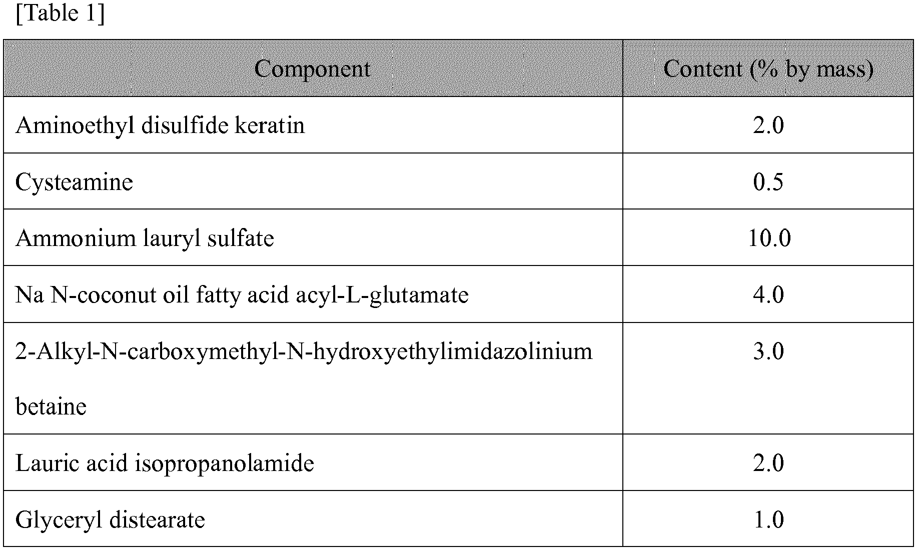

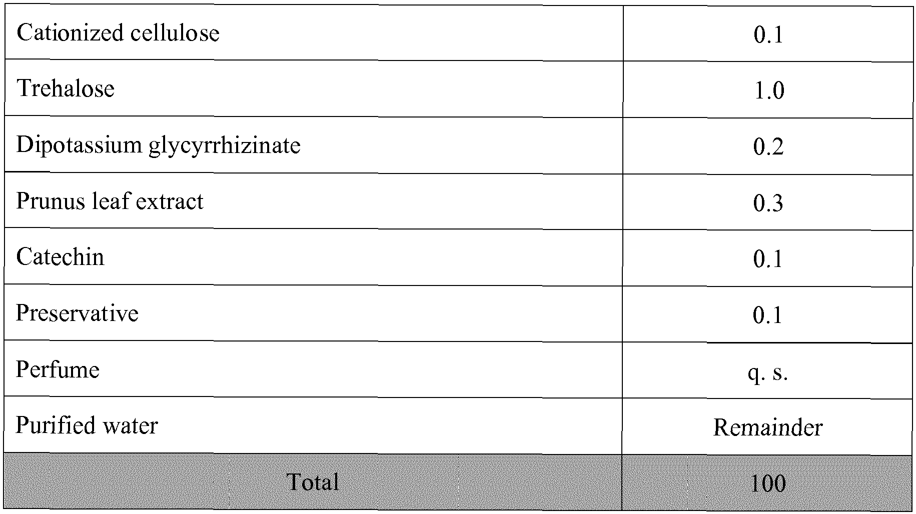

### (Formulation Example 2) Shampoo

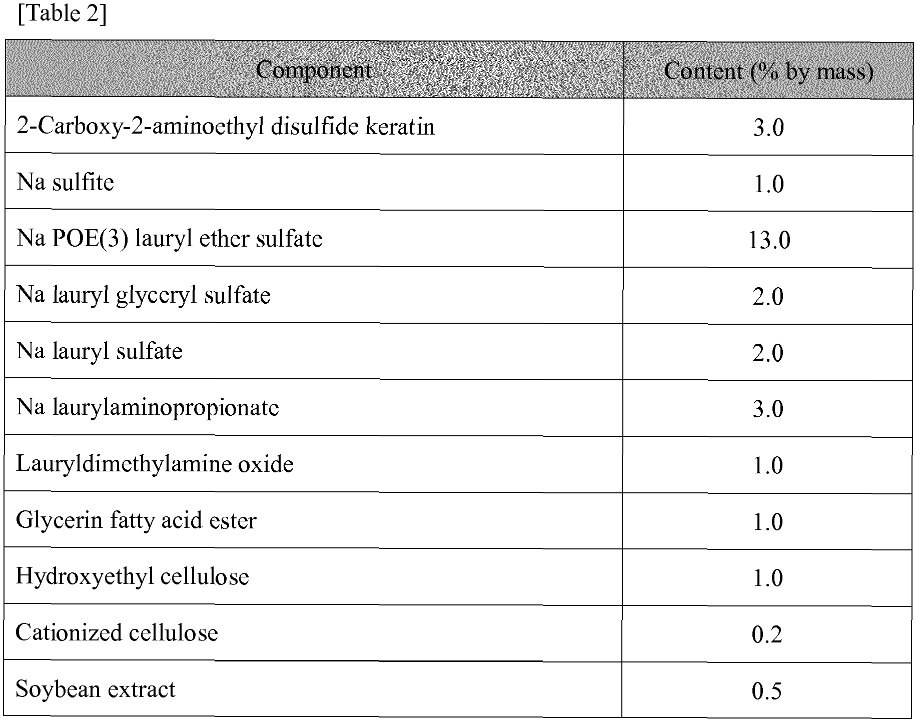

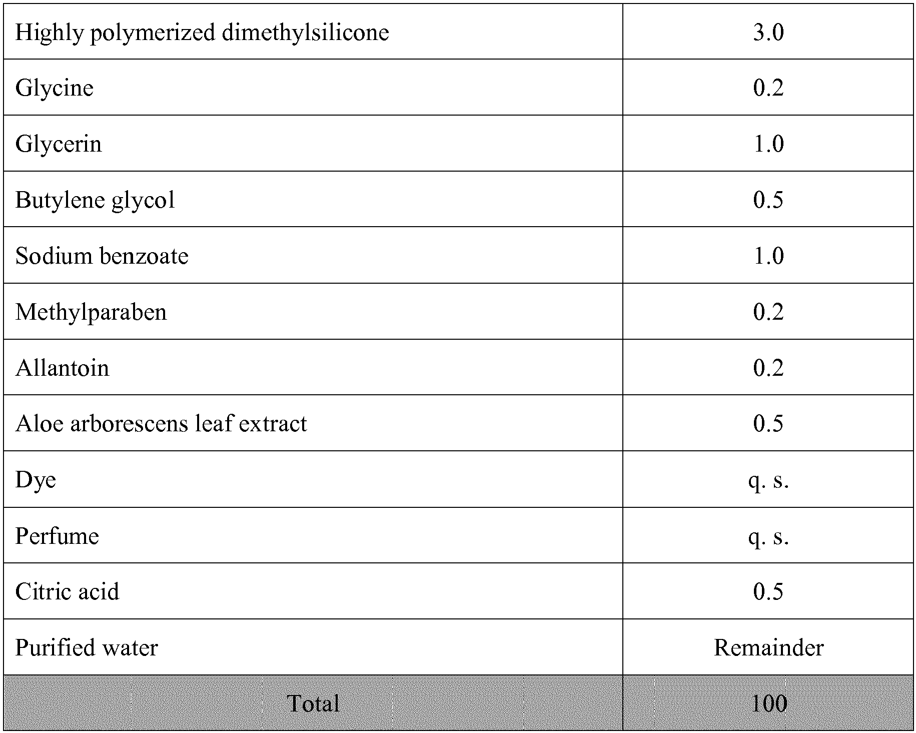

### (Formulation Example 3) Shampoo for animal

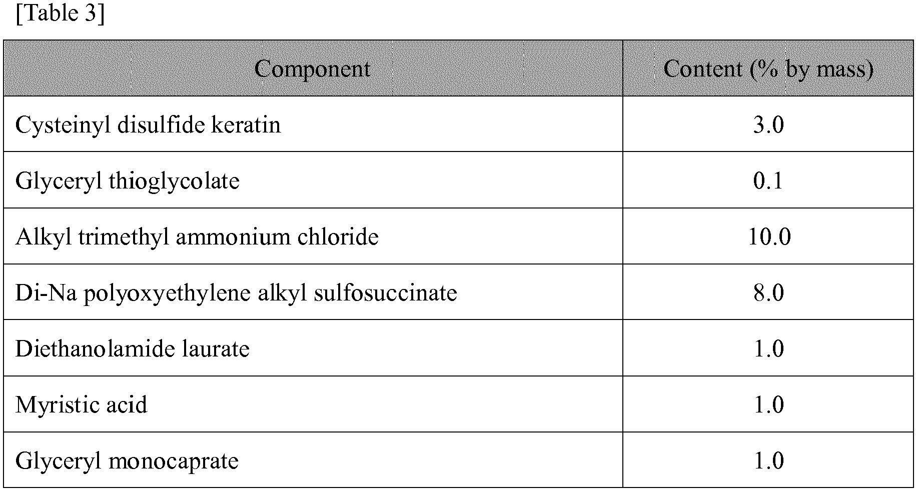

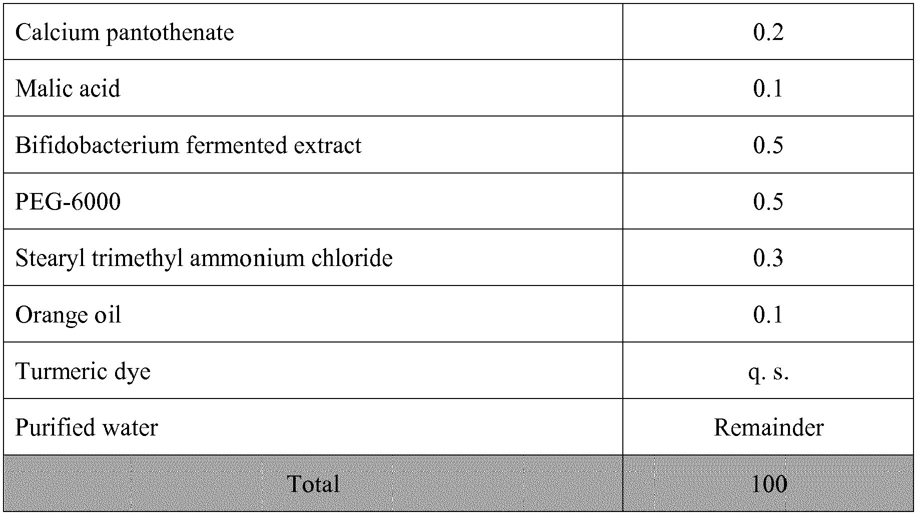

### (Formulation Example 4) Softener

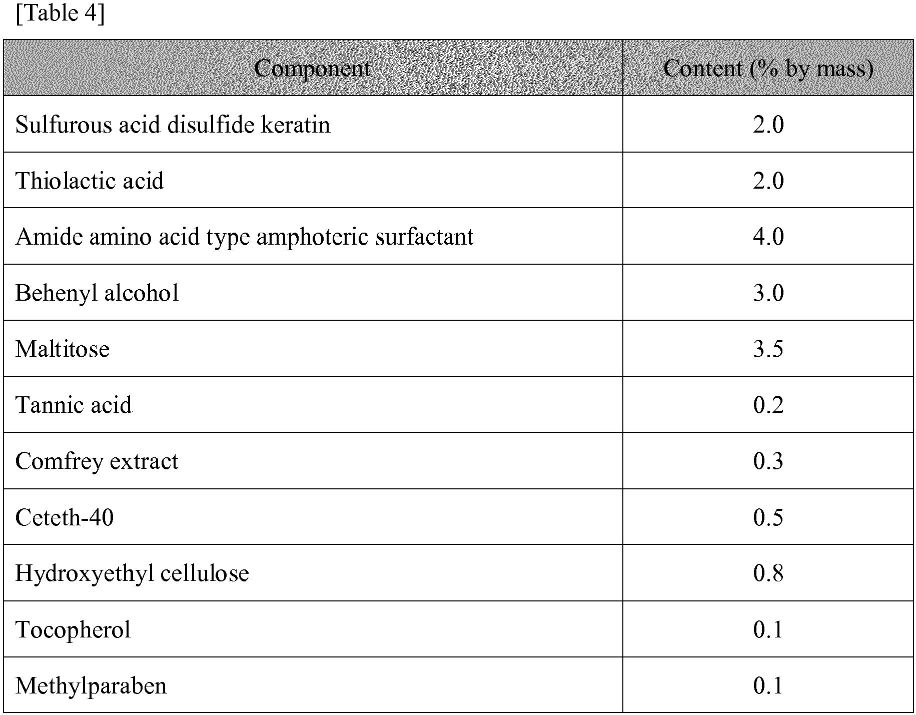

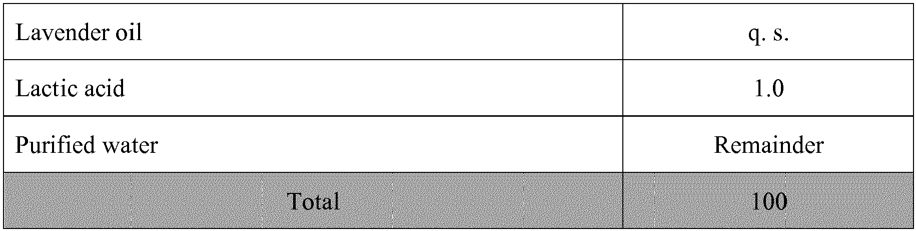

### (Formulation Example 5) Rinse for hair

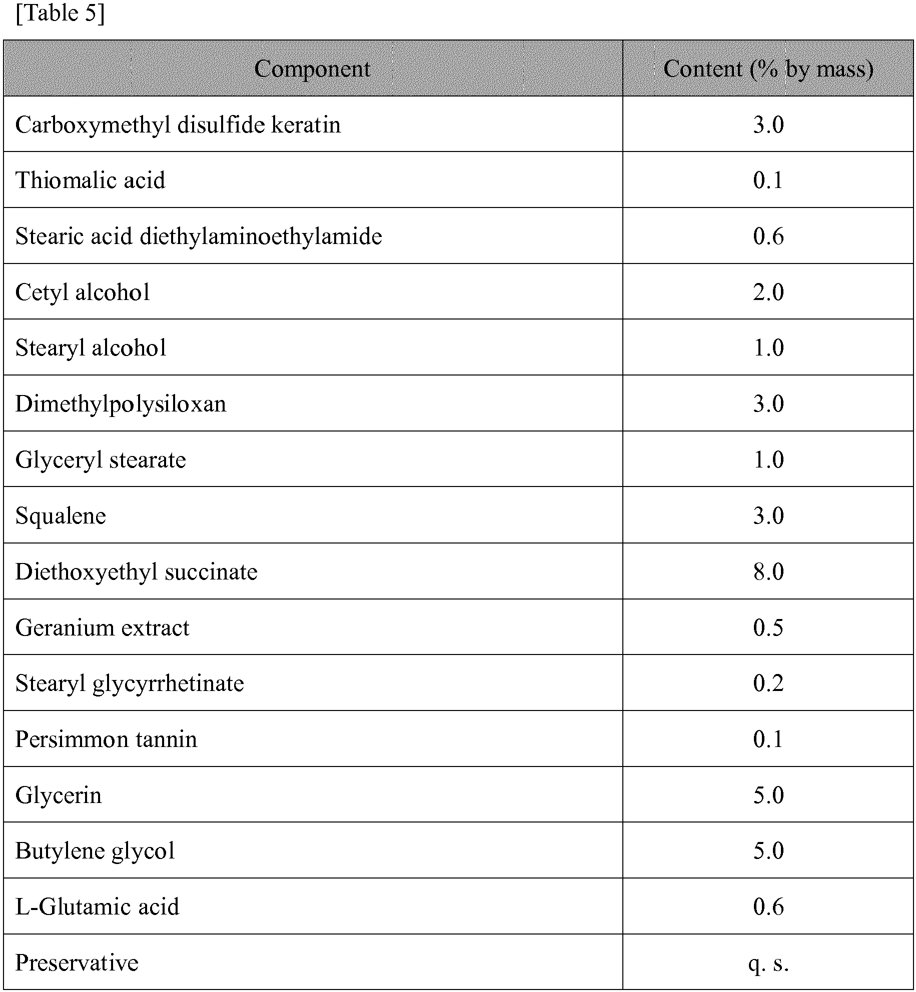

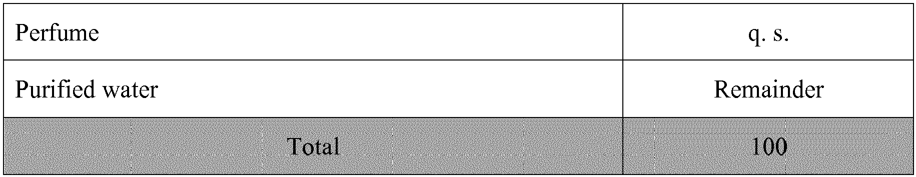

### (Formulation Example 6) Rinse for animal

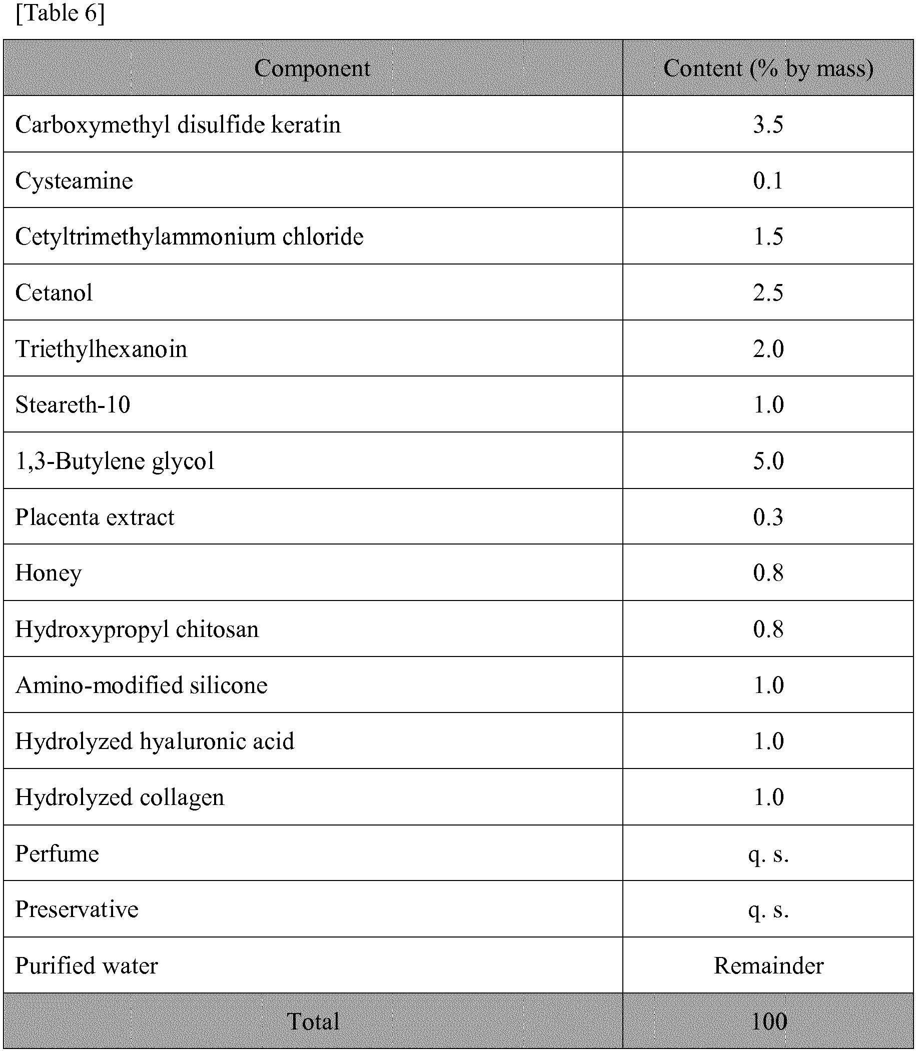

### (Formulation Example 7) Feel modifier

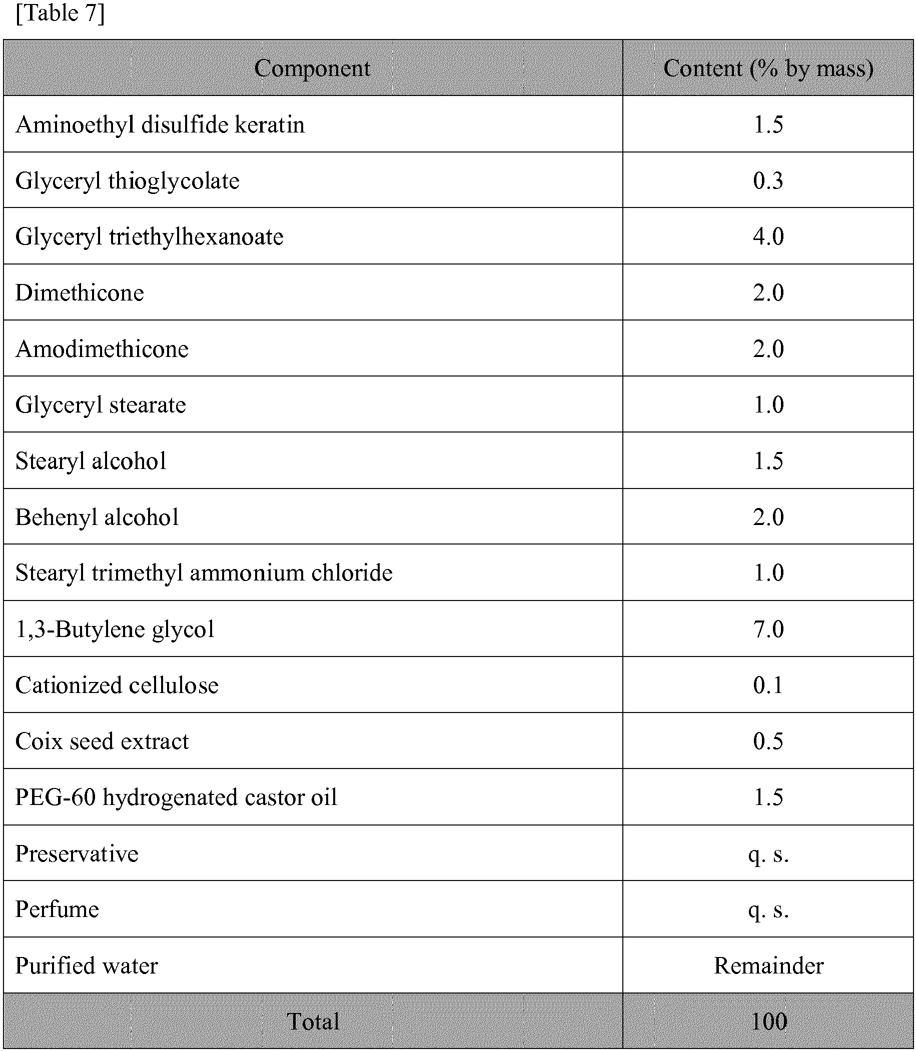

### (Formulation Example 8) Hair tonic

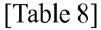

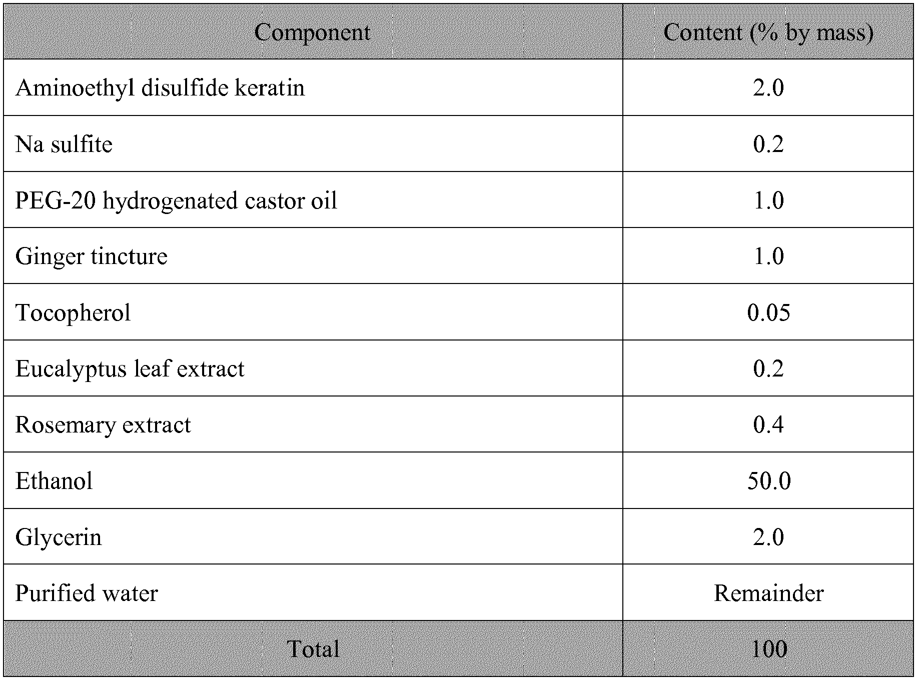

### (Formulation Example 9) Hair cream

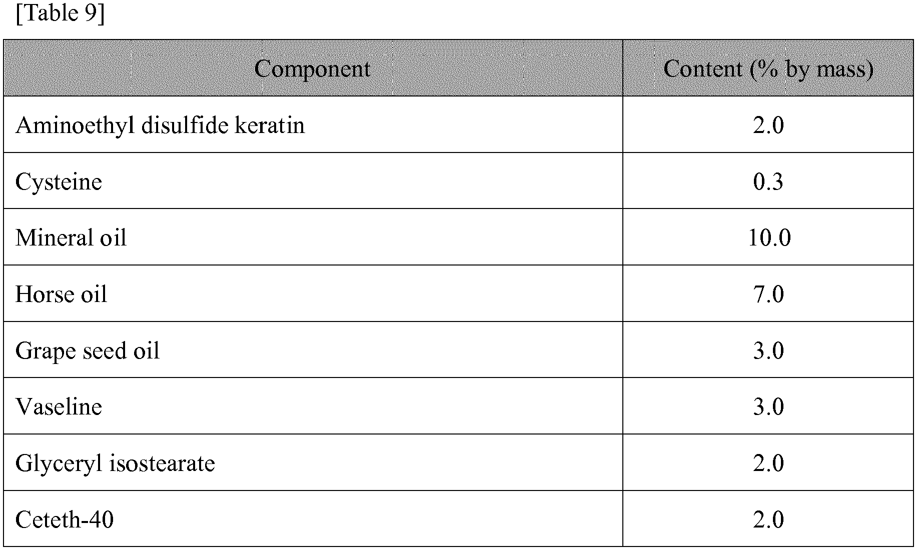

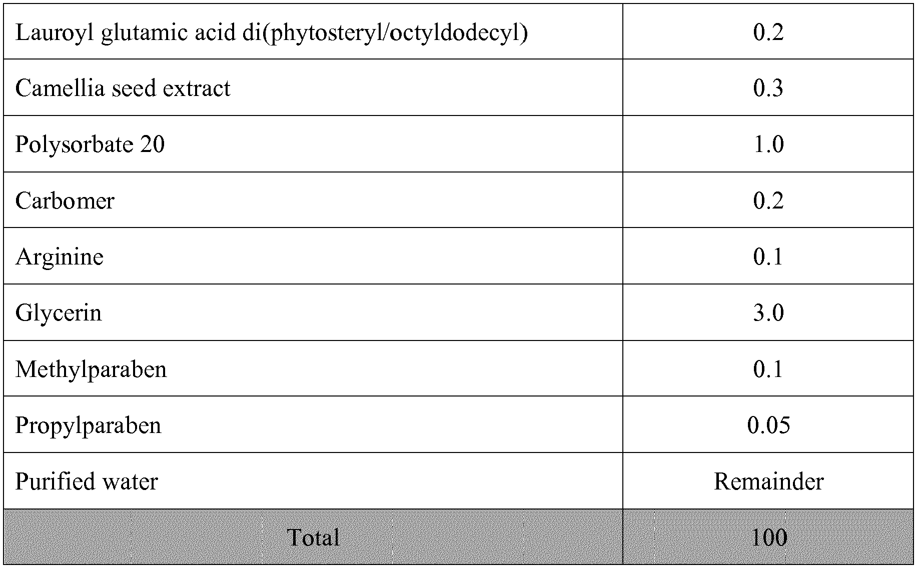

### (Formulation Example 10) Water repellent

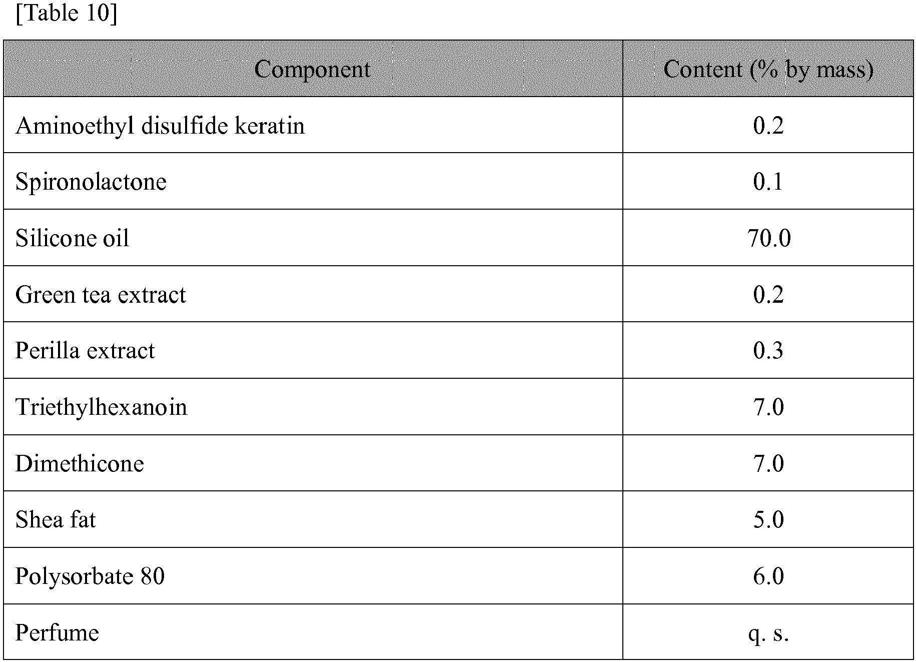

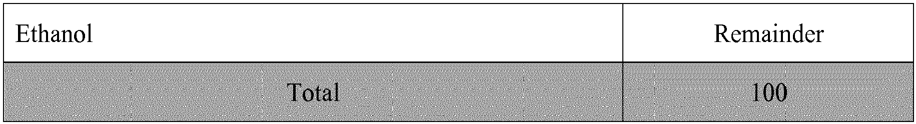

### (Formulation Example 11) Shape retention agent

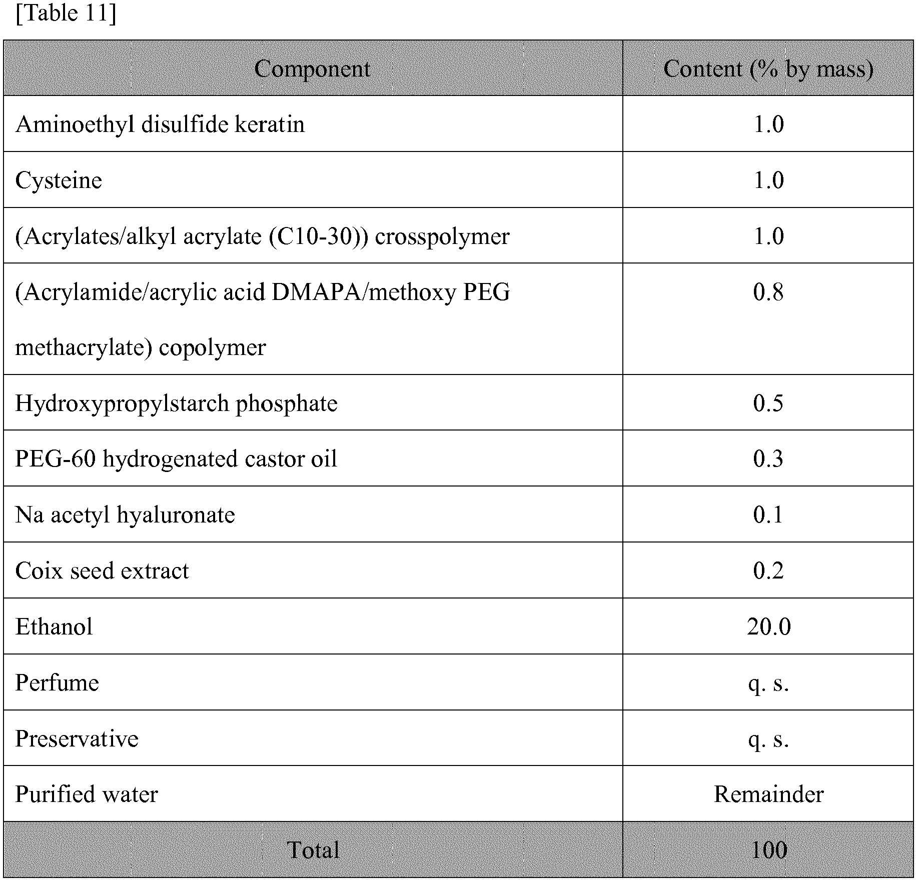

### (Formulation Example 12) Antistatic agent

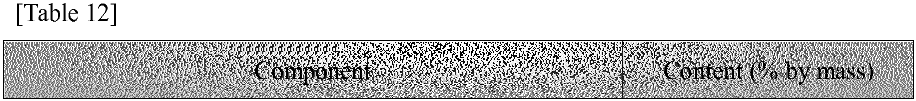

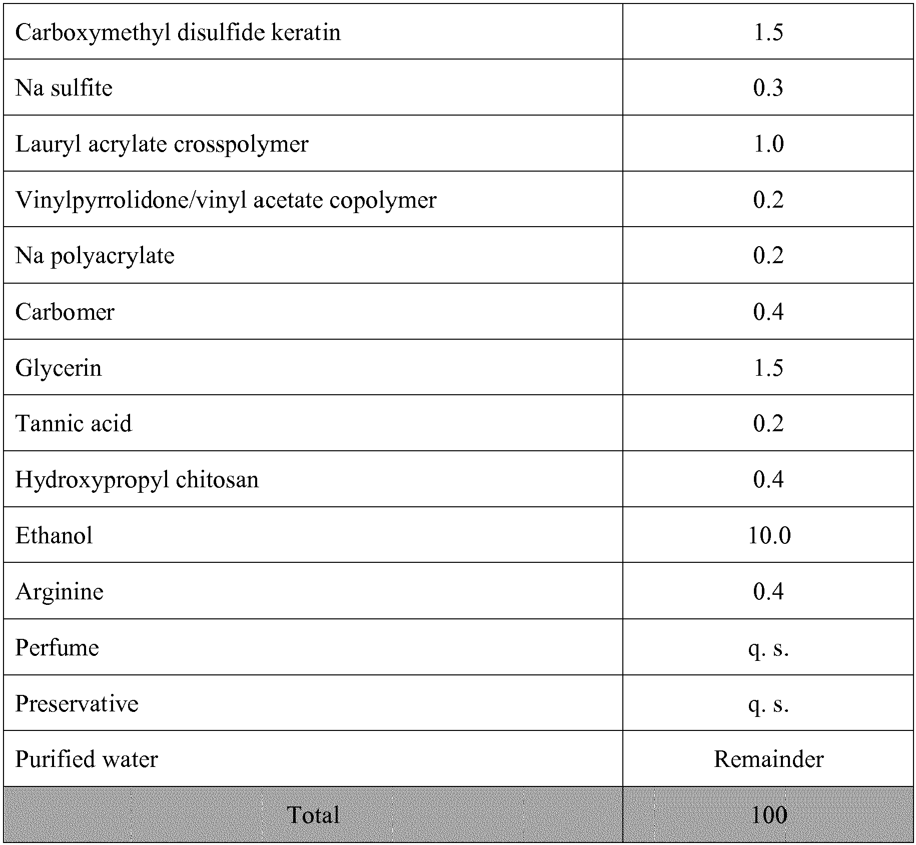

### (Formulation Example 13) Fiber repairing agent

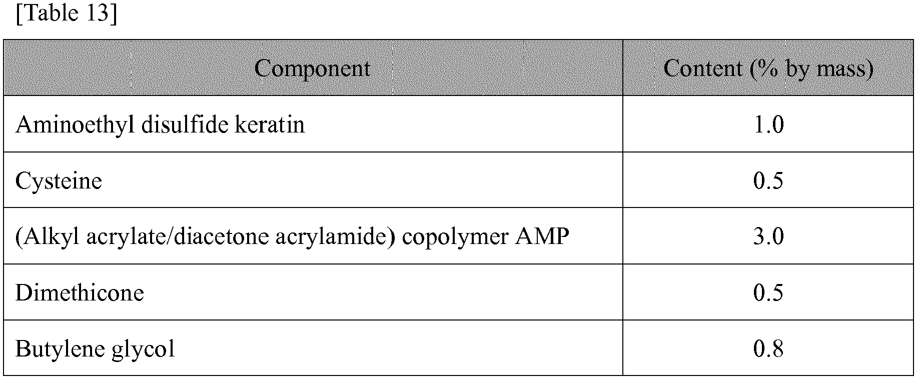

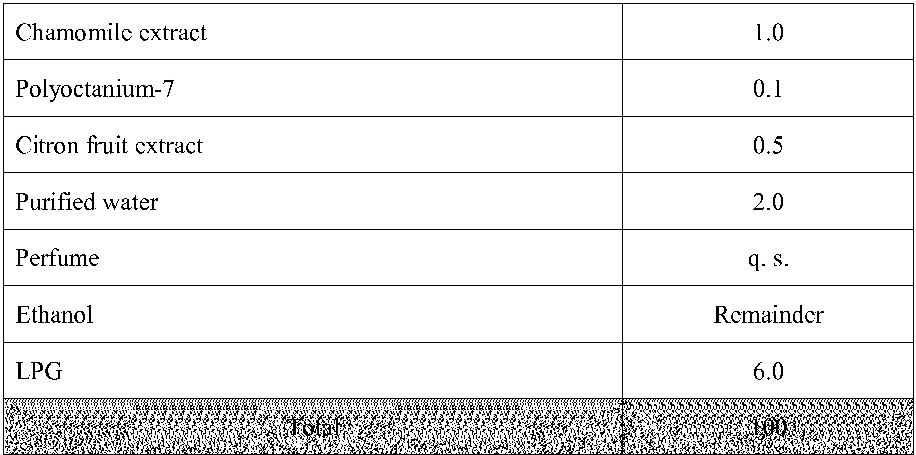

### (Formulation Example 14) Hair foam

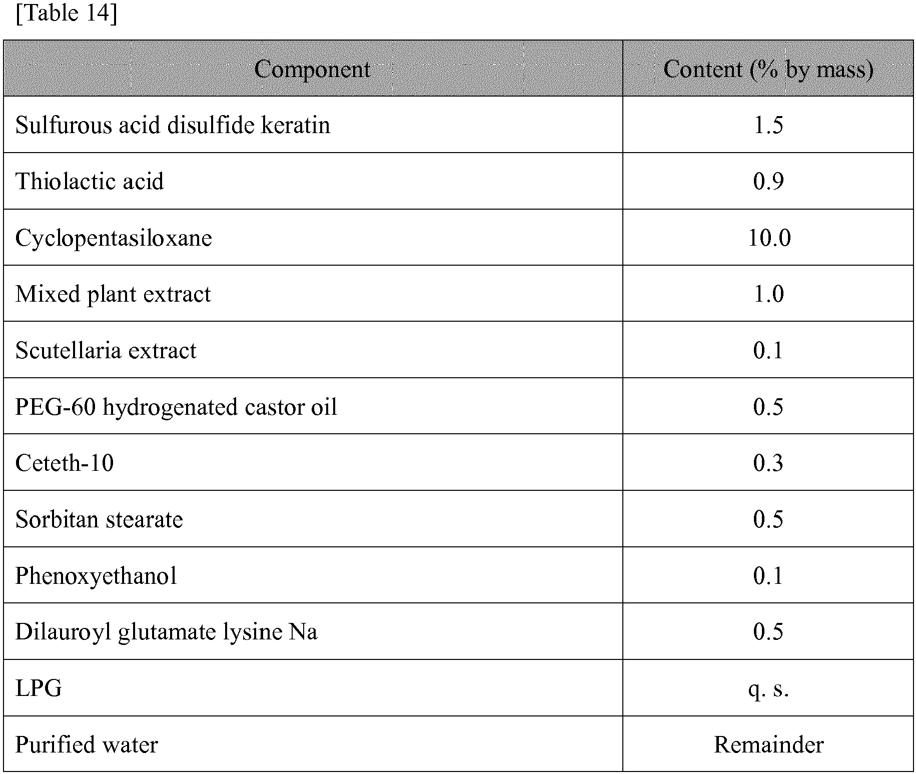

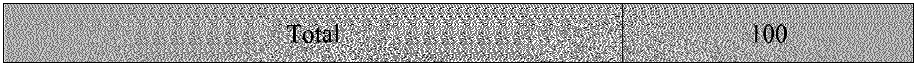

### (Formulation Example 15) Hair mist

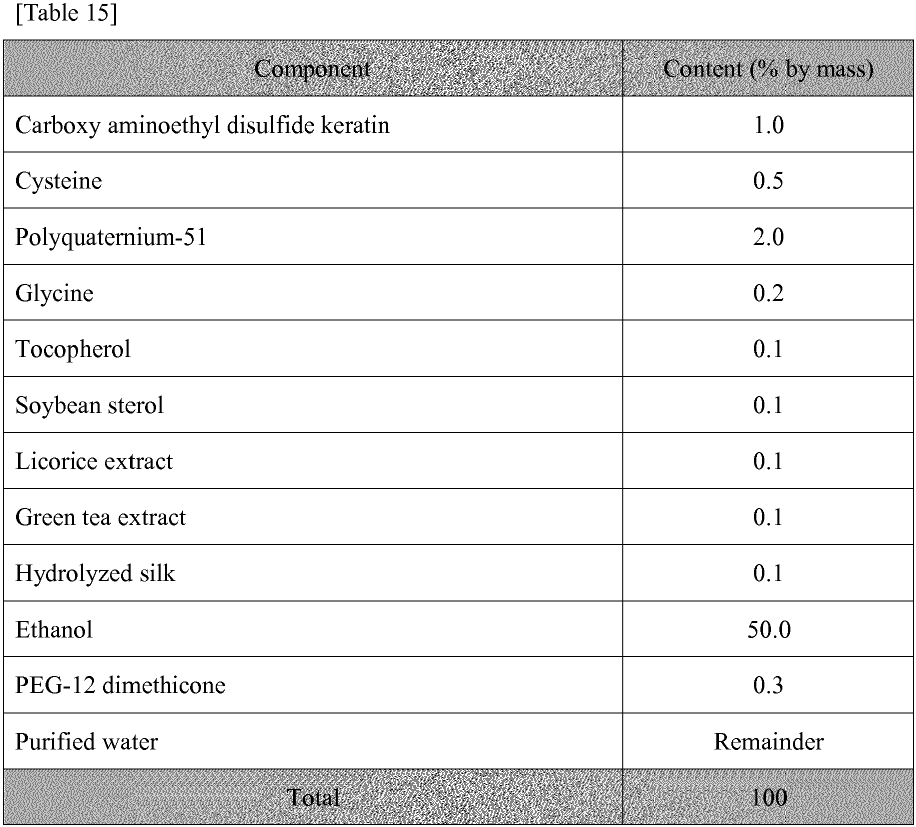

### (Formulation Example 16) Friction inhibitor

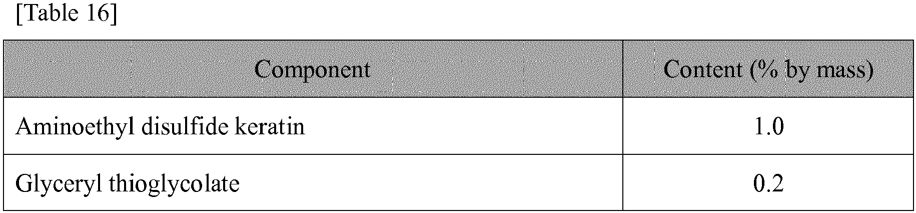

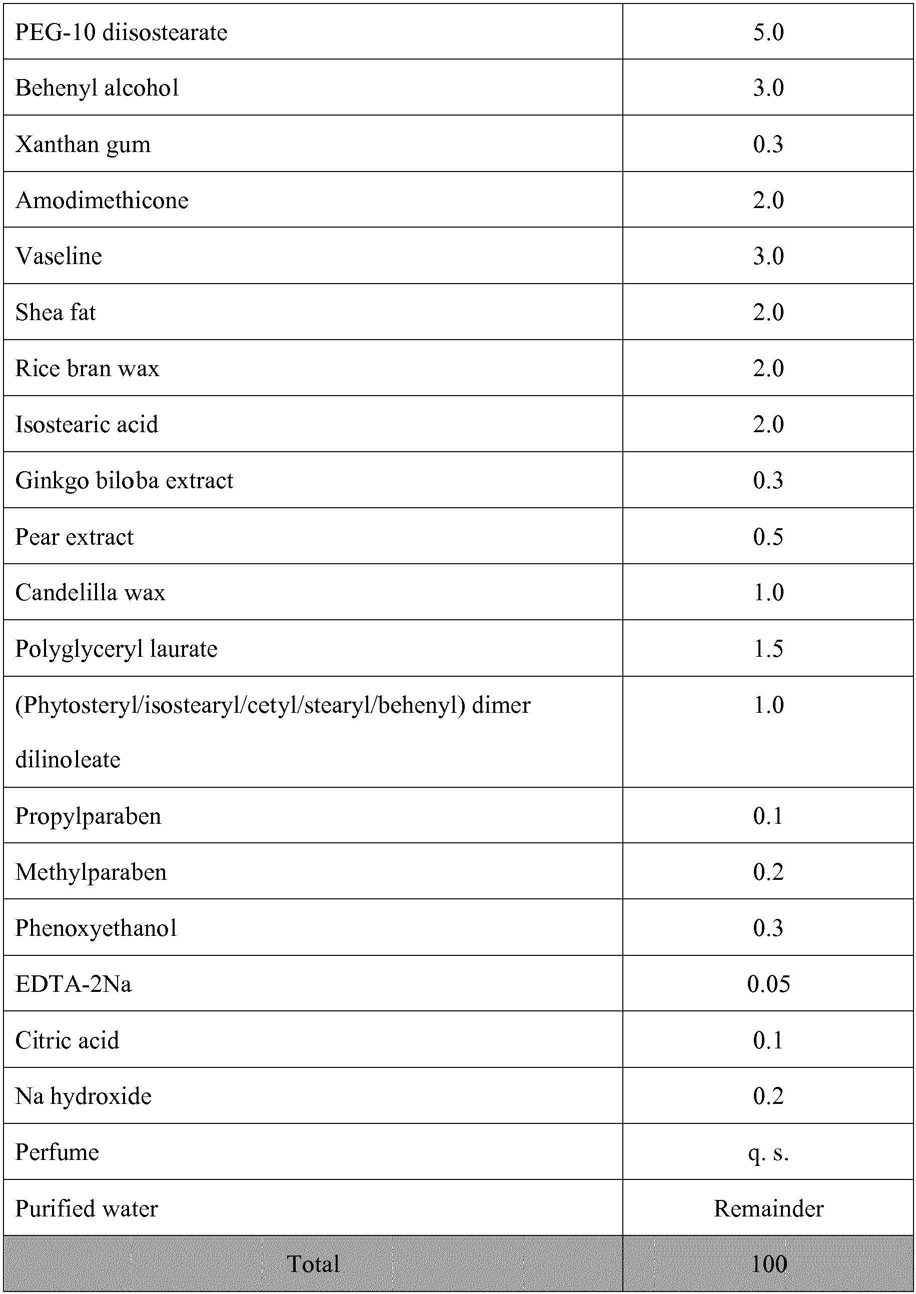

### (Formulation Example 17) Reactive hair dye

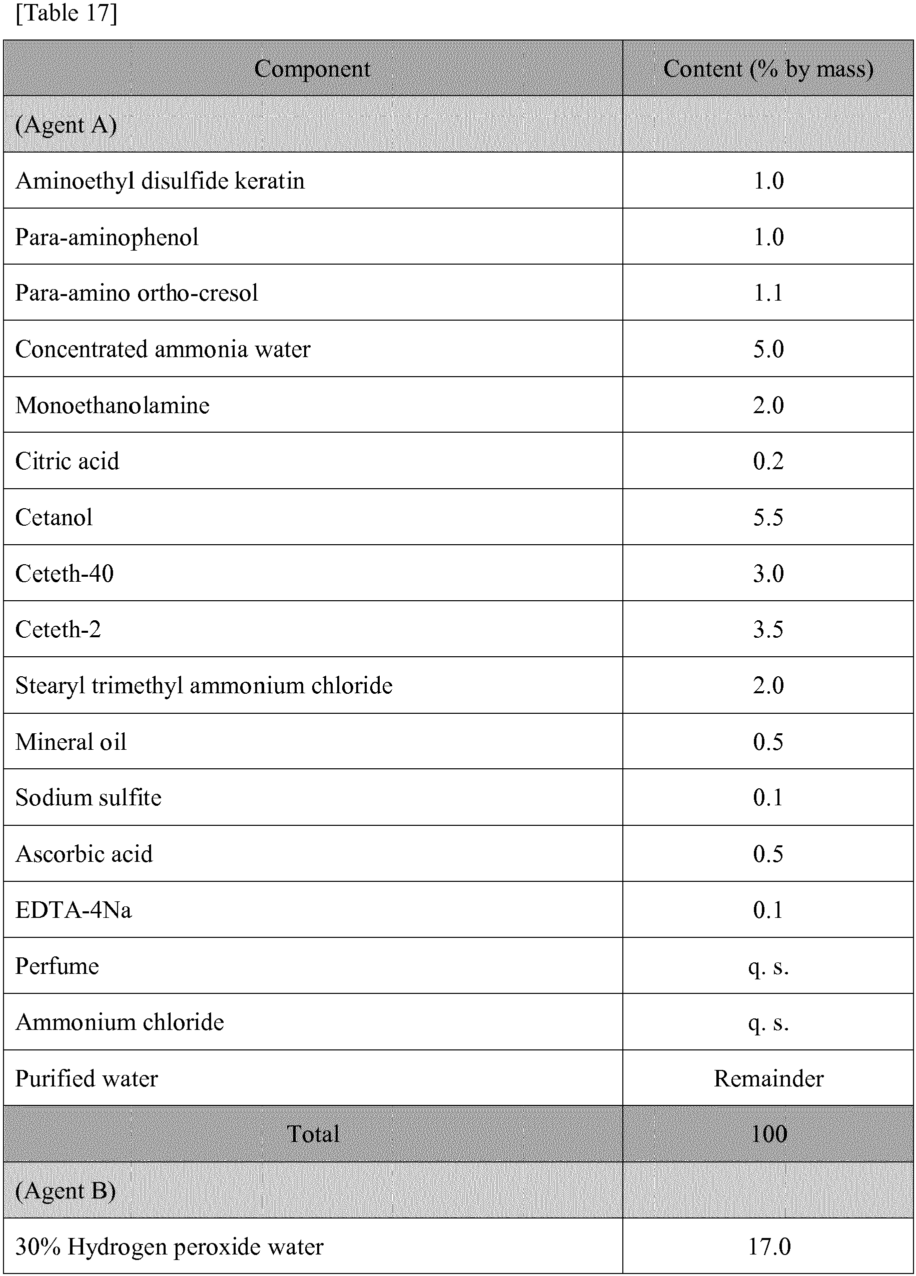

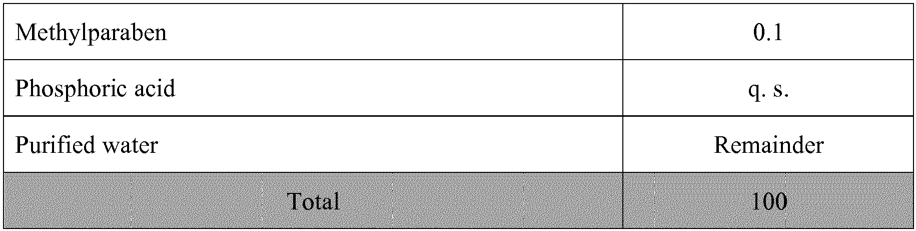

### (Formulation Example 18) Dyeing agent

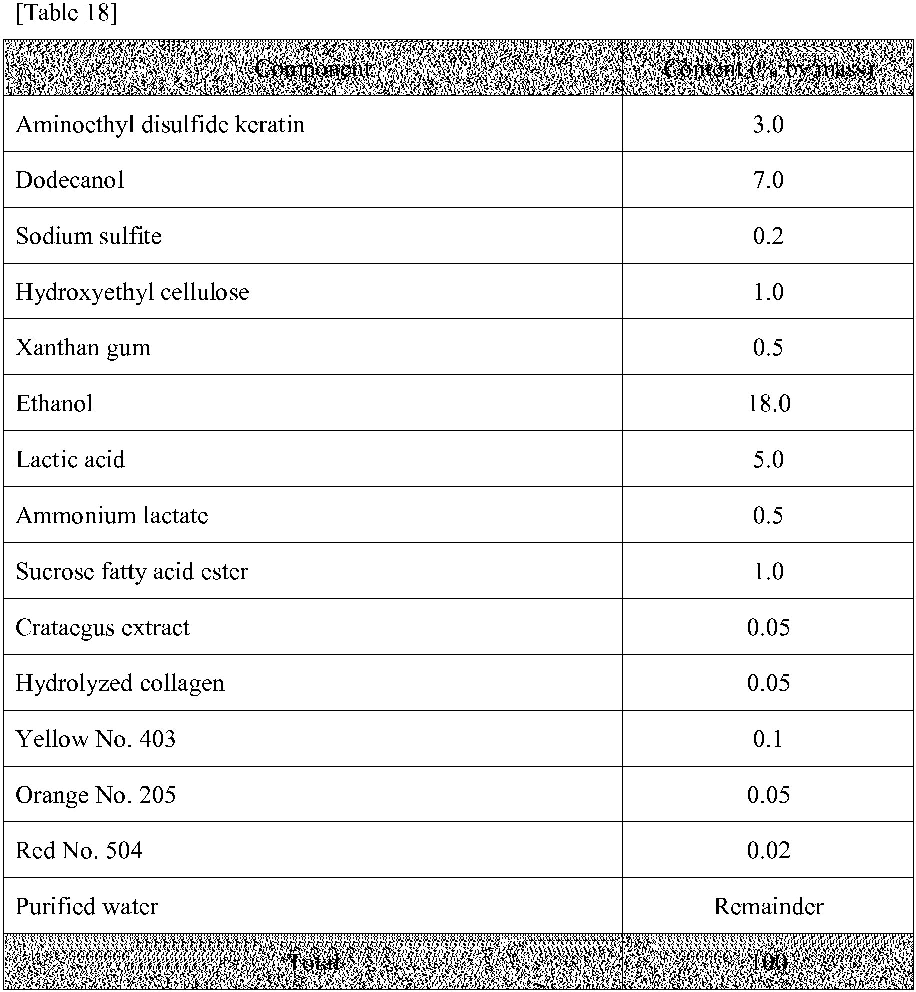

### (Formulation Example 19) Curling agent

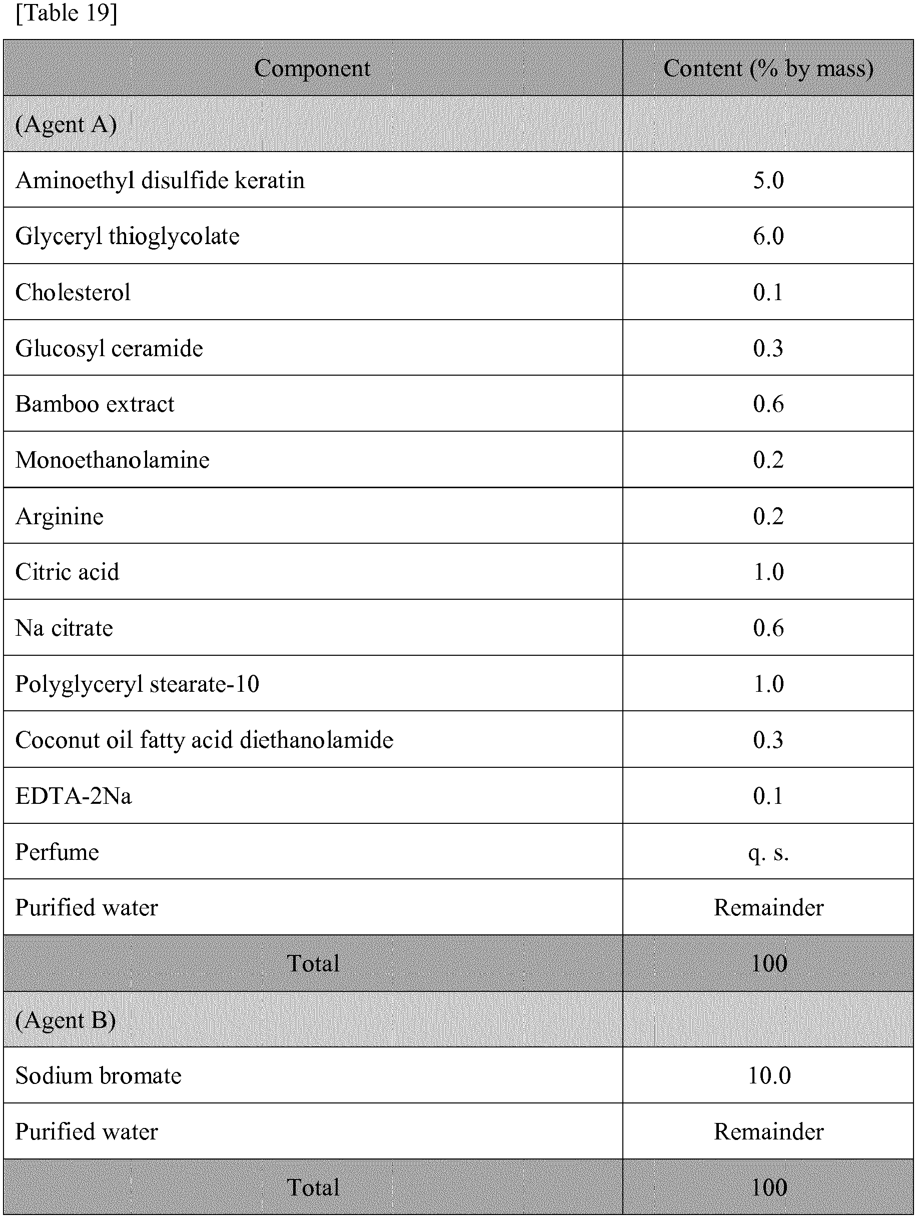

### (Formulation Example 20) Hair straightener

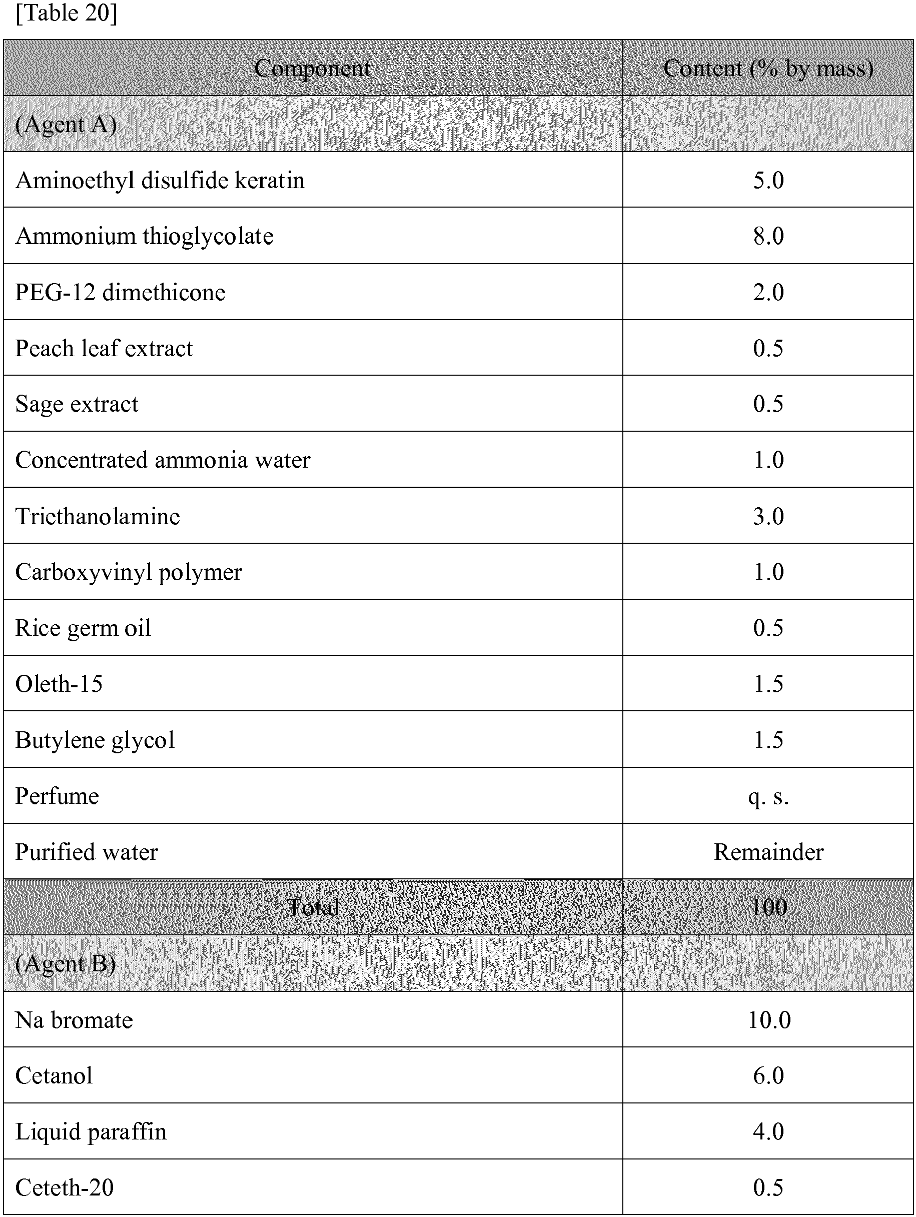

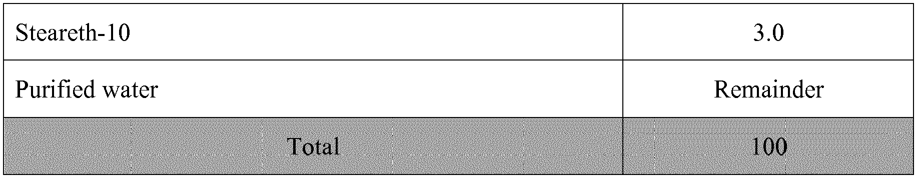

### (2) Evaluation Test 1

An evaluation test was performed using the curing agent in Formulation example 19. The agent A in Formulation example 19 was applied to hairs (one bundle 20 g, 20 cm) previously subjected to a medium bleaching treatment, and the applied hairs were allowed to stand for 15 minutes. After being sufficiently rinsed with water, the hairs were wiped with a towel to remove water contents. Next, the agent B in Formulation example 19 was applied to the hairs and the applied hairs were allowed to stand for 15 minutes for drying. As a control, the curing agents in Formulation example 19 without the converted protein or the reducing agent were produced, and hairs were treated with these curing agents in the same manner.

The strength of the hairs after the above treatment was measured using a digital force gauge manufactured by A&D Company, Ltd. A load was applied to one hair of each specimen and the force (unit: N) required for cutting the hair was determined as tensile strength. Note that 20 hairs were measured for each specimen and their average value was used for evaluation. The result is shown in Fig. 1.
Specimen 1: Medium-bleached hair (untreated).
Specimen 2: Medium-bleached hair treated with agent in Formulation example 19 without converted protein.
Specimen 3: Medium-bleached hair treated with agent in Formulation example 19 without reducing agent.
Specimen 4: Medium-bleach hair treated with Formulation example 19.
Specimen 5: Normal hair (no medium bleaching treatment).

The agent A in Formulation example 19 is the fiber treatment agent containing the converted protein and the reducing agent. The treatment by the agent A is a step for converting disulfide bonds contained in the keratin protein in a hair and the converted protein to thiols. The agent B in Formulation example 19 contains an oxidizing agent. The treatment by the agent B is a step for causing an oxidative reaction between the thiol of the hair and the thiol of the converted protein generated by the agent A and bonding them together.

The specimen 1, which was a medium-bleached hair, showed a significant reduction in the strength as compared with the specimen 5 (normal hair). The specimen 2 was subjected to the reduction and oxidation treatments without the converted protein, the treatments being the same as so-called permanent wave and curing agent treatments. The specimen 2, in which the treatments only caused a damage to a hair protein, showed the further lower hair strength than the specimen 1. The specimen 3, which was treated without the reducing agent, showed an improvement in the hair strength, however, an increase in the hair strength was found to be small.

In contrast, the specimen 4, which was treated with Formulation example 19 (one embodiment of the fiber treatment agent of the present invention), showed more improvement in the strength than the specimen 3 and recovered the strength to the same level as the normal hair. It is speculated the specimen 3 treated without the reducing agent showed only a slight increase in the strength due to insufficient adhesion of the converted protein to the hair. On the other hand, it is speculated the specimen 4 showed a significant increase in the strength due to sufficient adhesion of the converted protein to the hair (this description is merely an opinion of the inventor, not for defining the present invention, i.e., this description is by no means intended for defining the present invention).

### (2) Evaluation Test 2

A human hair was treated by the same method as that in the above Evaluation test 1 using the curing agent in Formulation example 19. Irregularity on a hair end on the hairs (so called ahoge) before and after the treatment was observed by visual inspection. The so called ahoge results from a leakage of a protein in a fiber from a cut surface of the fiber caused by various reasons and thus shows a low density of protein structure in the hair, making it difficult to maintain the original high dimensional structure of proteins.

In the hair after the treatment, irregularity on a hair end (so called ahoge) caused by flipping up or waviness was reduced by visual observation.

The treatment using the curing agent in Formulation example 19 can achieve excellent adhesion to an application site by converting a disulfide bond contained in the converted protein to a thiol group by the reducing agent and then oxidatively bonding the thiol group of the converted protein to a thiol group derived from a disulfide bond inside a fiber. It is speculated that this treatment makes it possible to improve the strength of a hair end of a fiber in which a density of proteins is reduced and a fiber damaged by various reasons, or improve irregularity on a hair end caused by a flipped-up or wavy fiber (this description is merely an opinion of the inventor, not for defining the present invention, i.e., this description is by no means intended for defining the present invention).

## Claims

1. A fiber treatment agent, wherein:
the fiber treatment agent is prepared in a one-component formulation containing an effective amount of a reducing agent and an effective amount of a converted protein in which some or all of disulfide bonds (-S-S-) in a protein are converted by at least one of a thiol compound selected from the group consisting of cysteamine, cysteine, and thioglycolic acid or sulfurous acid, or prepared in a two-component formulation including a first agent containing the converted protein and a second agent containing the reducing agent, in which a total content of the converted proteins and a total content of the reducing agents in the first agent and the second agent satisfy the respective effective amounts; and
the fiber treatment agent is used for improving strength of a fiber or improving irregularity on a hair end caused by a flipped-up or wavy fiber.

2. The fiber treatment agent according to claim 1, wherein the protein is keratin.

3. The fiber treatment agent according to claim 1 or 2, wherein the reducing agent is selected from the group consisting of at least one of thioglycolic acid, thiolactic acid, thiomalic acid, sulfurous acid, cysteamine, cysteine, spironolactone, and a salt or ester thereof.

4. The fiber treatment agent according to any one of claims 1 to 3, wherein the fiber treatment agent is a shampoo, a rinse, a treatment, a conditioner, a hair oil, a hairdressing agent, a permanent waving agent, a curling agent, a hair coloring agent, or a cosmetic used for a treatment before or after using these agents.

5. A method for using the fiber treatment agent, wherein the fiber treatment agent according to any one of claims 1 to 4 is applied to a fiber and then an oxidizing agent is applied to the fiber.

6. The method according to claim 5, wherein the fiber is a hair.
